# DEMANDE DE BREVET EUROPEEN

(11) **EP 0 963 981 A1**
(43) Date de publication de la demande: **15.12.1999**
(21) Numéro de dépôt: 99401251.6
(22) Date de dépôt: 25.05.1999
(51) Int. Cl.: C07D 307/79, C07D 333/54, C07D 307/80, C07D 333/56, C07D 209/08, C07D 409/04, A61K 31/34

(54) **Composés biaryles hétérocycliques aromatiques, compositions pharmaceutiques et cosmétiques les contenant et utilisations**

(30) Priorité: 12.06.1998 FR 9807438
(71) Demandeur: Galderma Research & Development, S.N.C., 06560 Valbonne (FR)
(72) Inventeur: Charpentier, Bruno, 06410 Biot (FR); Nedoncelle, Philippe, 06130 Grasse (FR)
(74) Mandataire: Tezier Herman, Béatrice

(57) **Abrégé**

L'invention concerne de nouveaux composés biarylhétérocycliques aromatiques qui présentent comme formule générale (I): 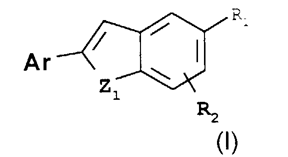 ainsi que l'utilisation de ces derniers dans des compositions pharmaceutiques destinées à un usage en médecine humaine ou vétérinaire (affections dermatologiques, rhumatismales, respiratoires, cardiovasculaires et ophtalmologiques notamment), ou bien encore dans des compositions cosmétiques.

## Description

L'invention concerne, à titre de produits industriels nouveaux et utiles, des composés biarylhétérocycliques aromatiques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire et trouvent des applications plus particulièrement dans le traitement topique et systémique des affections dermatologiques(ou autres) liées à un désordre de la kératinisation, des affections à composante inflammatoire et/ou immunoallergique et de l'hyperprolifération des tissus d'origine ectodermique (peau, épithélium...), qu'elle soit bénigne ou maligne. Ces composés peuvent en outre être utilisés dans le traitement de maladies de dégénérescence du tissu conjonctif, pour lutter contre le vieillissement de la peau, qu'il soit photoinduit ou chronologique et traiter les troubles de la cicatrisation. Ils trouvent notamment une application en ophtalmologie dans le traitement des cornéopathies.
On peut également utiliser les composés selon l'invention dans des compositions cosmétiques pour l'hygiène corporelle et capillaire.

La présente invention concerne des composés qui peuvent être représentés par la formule générale (I) suivante : 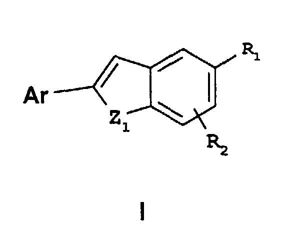 dans laquelle,
Ar représente un radical choisi parmi les radicaux de formules (II)-(IV) suivantes : 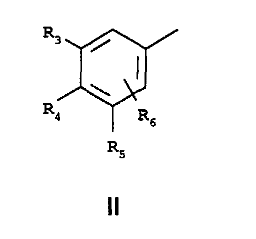 R₃, R₄, R₅, et R₆ ayant les significations données ci-après, 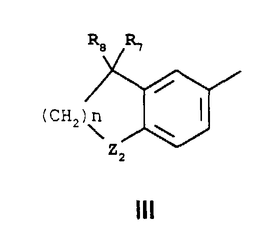 n, Z₂, R₇ et R₈ ayant les significations données ci-après, 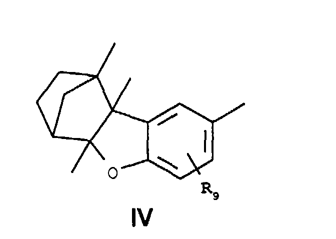 R₉ ayant la signification donnée ci-après,
Z₁ représente un atome d'oxygène, de soufre ou le radical NR',
   R' ayant la signification donnée ci-après,
Z₂ représente C(R₇R₈), O, NR', S, SO, ou SO₂,
   R₇, R₈, R' ayant les significations données ci-après,
R₁ représente
   (i) un radical -CH₃,
   (ii) un radical -(CH₂)ₘ-O-R_{10,}
   (iii) un radical -CH₂-O-CO-R₁₁
   (iv) un radical -(CH₂)ₓ-CO-R₁₂
   (v) un radical -(CH₂)ₓ-CO-OR₁₃
   R₁₀, R₁₁, R₁₂ et R₁₃, m, x et t ayant les significations données ci-après,
R₂ représente un atome d'hydrogène, un atome d'halogène, un radical alkoxy linéaire ou ramifié de 1 à 20 atomes de carbone, ou un radical -O-CH₂-O-CH₂-CH₂-O-CH₃,
R₃ et R₅ identiques ou différents représentent un atome d'hydrogène, un radical alkyle, ou un radical cycloalkyle,
   avec les conditions suivantes :
   - R₃ et R₅ ne représentent pas simultanément un atome d'hydrogène,
   - lorsque R₃ ou R₅ représente un radical adamantyl alors Z₁ est différent d'un atome d'oxygène,
R₄ représente un radical -O-CH₂-O-CH₂-CH₂-O-CH₃, un radical -(Y)_{q}-(CH₂)ₛ-R₁₄, un radical -(CH₂)_{z}-Y-(CH₂)ₛ-R₁₄, ou un radical -CH=CH-(CH₂)_{w}-R₁₄,
   Y et R₁₄ ayant les significations données ci-après,
   q, z, s, w, identiques ou différents ayant les significations données ci-après,
R₆ représente un atome d'hydrogène, un atome d'halogène, un radical alkoxy linéaire ou ramifié de 1 à 20 atomes de carbone ou un radical -O-CH₂-O-CH₂-CH₂-O-CH₃,
R₇ et R₈ identiques ou différents représentent un radical alkyle inférieur,
R₉ représente un atome d'hydrogène, un atome d'halogène, un radical alkoxy linéaire ou ramifié de 1 à 20 atomes de carbone, ou un radical -O-CH₂-O-CH₂-CH₂-O-CH₃,
R₁₀ représente un hydrogène ou un radical alkyle inférieur,
R₁₁ représente un radical alkyle inférieur,
R₁₂ représente un atome d'hydrogène, un radical alkyle inférieur ou un radical -N(R",R"'),
   R" et R"' ayant les significations données ci-après,
R₁₃ représente un atome d'hydrogène, un radical alkyle linéaire ou ramifié de 1 à 20 atomes de carbone, un radical alkényle, un radical mono- ou polyhydroxyalkyle,
R₁₄ représente un radical choisi parmi :
   (i) un atome d'hydrogène,
   (ii) un radical alkyle inférieur,
   (iii) un radical alkényle,
   (iv) un radical alkynyle,
   (v) un radical cycloaliphatique ayant de 3 à 6 atomes de carbone,
   (vi) un radical mono ou polyhydroxyalkyle, les groupements hydroxyles pouvant optionnellement être protégés sous forme de méthoxy, acétoxy ou acétonide,
   (vii) un radical CO-R₁₂,
   (viii) un radical COO-R₁₃,
   (ix) un radical hydroxyle, un radical O-R₁₅ ou O-CO-R₁₅, à la condition que R₄ représente le radical -(Y)_{q}-(CH₂)ₛ-R₁₄ avec q est égal à 0,
      Y, et R₁₅ ayant les significations données ci-après,
      q et s ayant les significations données ci-après,
R₁₅ représente un radical alkyle inférieur,
R' représente un atome d'hydrogène, un radical alkyle inférieur ou un groupement protecteur de la fonction amine,
R" et R"' identiques ou différents, représentent un atome d'hydrogène, un radical alkyle inférieur, un radical mono ou polyhydroxyalkyle, ou encore, R" et R"' pris ensemble, forment un hétérocycle,
Y représente S, O ou S(O)t,
   t ayant la signification donnée ci-après,
   m représente un nombre entier pouvant prendre une valeur allant de 0 à 2,
   n représente un nombre entier pouvant prendre la valeur 1 ou 2,
   q représente un nombre entier pouvant prendre la valeur 0 ou 1,
   s représente un nombre entier pouvant prendre une valeur entière allant de 0 à 12,
   t représente un nombre entier pouvant prendre une valeur allant de 0 à 3,
   w représente un nombre entier pouvant prendre une valeur entière allant de 0 à 10,
   x représente un nombre entier pouvant prendre une valeur allant de 0 à 2,
   z représente un nombre entier pouvant prendre la valeur 1, 2 ou 3,

L'invention vise également les isomères optiques ou géométriques desdits composés de formule (I) ainsi que leurs sels.

Lorsque les composés selon l'invention se présentent sous forme de sels par addition d'une base, il s'agit de sels d'un métal alcalin ou alcalino-terreux ou encore de zinc ou d'une amine organique.

Lorsque les composés se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique.

Selon la présente invention, on entend par radical alkyle inférieur un radical linéaire ou ramifié ayant de 1 à 6 atomes de carbone et, de préférence, les radicaux méthyle, éthyle, isopropyle, n-butyle et tertiobutyle.

Par radical alkyle on entend un radical linéaire ou ramifié ayant de 1 à 20 atomes de carbone éventuellement substitué par un ou plusieurs atomes d'halogène et de préférence les radicaux méthyle, éthyle, isopropyle, butyle, tertiobutyle et hexyle.

Par radical alkényle on entend un radical linéaire ou ramifié ayant de 1 à 20 atomes de carbone comportant une ou plusieurs doubles liaisons.

Par radical alkynyle on entend un radical linéaire ou ramifié ayant de 1 à 20 atomes de carbone comportant une à plusieurs triple liaisons.
Parmi les atomes d'halogène, on préfère un atome de fluor, de chlore ou de brome.

Par groupement protecteur de fonction amine, on entend les groupements correspondants décrits dans "Protecting groups in organic synthesis" by T.W Greene, Ed. by John Wiley and Sons (1981), et, de préférence un groupement formamide, acétamide, chloracétamide, trifluoroacétamide ou benzyloxycarbonyle.

Par radical cycloalkyle, on entend un radical alcane cyclique ou polycyclique contenant de 1 à 10 atomes de carbone, éventuellement substitué par :
- un ou plusieurs atomes d'halogène,
et/ou
- un plusieurs radicaux hydroxyles,
De préférence le radical cycloalkyle est choisi parmi un radical adamantyle ou un radical 1-méthylcyclohexyle.

Par radical monohydroxyalkyle, on entend un radical ayant de 1 à 6 atomes de carbone, notamment un radical 2-hydroxyéthyle, 2-hydroxypropyle ou 3-hydroxypropyle.

Parmi les radicaux alkoxy, linéaire ou ramifié de 1 à 20 atomes de carbone on préfère les radicaux de 1 à 9 atomes de carbone, notamment les radicaux méthoxy, propyloxy, pentyloxy, heptyloxy.

Par radical polyhydroxyalkyle, on entend un radical contenant de 1 à 6 atomes de carbone et de 1 à 5 groupes hydroxyles, tels que les radicaux 2,3-dihydroxypropyle, 2,3,4-trihydroxybutyle, 2,3,4,5-tétrahydroxypentyle ou un résidu de pentaérythritol.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Par radical cycloaliphatique ayant de 3 à 6 atomes de carbone, on entend de préférence un radical cyclopropyle ou un radical cyclohexyle.

Parmi les composés entrant dans le cadre de la présente invention, on peut notamment citer les suivants :
- 2-(5,6,7,8-tetrahydro-5,5,8,8,-tetramethyl-2-naphthyl)-5-benzo(b)furanne carboxylate de méthyle,
- Acide 2-(5,6,7,8-tetrahydro-5,5,8,8,-tetramethyl-2-naphthyl)-5-benzo(b)furanne carboxylique,
- 2-(5,6,7,8-tetrahydro-5,5,8,8,-tetramethyl-2-naphthyl)-5-benzo(b)thiophene carboxylate de methyle,
- Acide 2-(5,6,7,8-tetrahydro-5,5,8,8,-tetramethyl-2-naphthyl)-5-benzo(b)thiophene carboxylique,
- Acide 2-(5,6,7,8-tetrahydro-5,5,8,8,-tetramethyl-2-naphthyl)-5-indole carboxylique,
- 2-(5,6,7,8-tetrahydro-5,5,8,8,-tetramethyl-2-naphthyl)-5-indole carboxylate de methyle,
- Acide 2-(1,2,3,4,4a,9b-Hexahydro-1,4a,9b-triméthyl-1,4-méthanodibenzofuran-8-yl)-benzo(b)thiophene-5-carboxylique,
- 2-(1,2,3,4,4a,9b-Hexahydro-1,4a,9b-triméthyl-1,4-méthanodibenzofuran-8-yl)-benzo(b)thiophene-5-carboxylate de methyle,
- Acide 2-(1,2,3,4,4a,9b-Hexahydro-1,4a,9b-triméthyl-1,4-méthanodibenzofuran-8-yl)-benzo(b)furanne-5-carboxylique ;
- Acide 2-(5,6,7,8-tetrahydro-5,5,8,8,-tetramethyl-3-methoxy-2-naphtyl)-5-benzo(b)furanne carboxylique,
- Acide 2-(5,6,7,8-tetrahydro-5,5,8,8,-tetramethyl-3-methoxy-2-naphtyl)-5-benzo(b)thiophene carboxylique,
- Acide 2-(5,6,7,8-tetrahydro-5,5,8,8,-tetramethyl-3-n-propyloxy-2-naphtyl)-5-benzo(b)thiophene carboxylique,
- Acide 2-(5,6,7,8-tetrahydro-5,5,8,8,-tetramethyl-3-n-propyloxy-2-naphtyl)-5-benzo(b)furanne carboxylique,
- Acide 2-(5,6,7,8-tetrahydro-5,5,8,8,-tetramethyl-3-n-heptyloxy-2-naphtyl)-5-benzo(b)furanne carboxylique,

Selon la présente invention, les composés de formule (I) plus particulièrement préférés sont ceux pour lesquels au moins l'une des, et de préférence toutes les, conditions suivantes sont remplies:
- R₁ est un radical -(CH₂)ₓ-CO-R₁₂ ou -(CH₂)ₓ-CO-O-R₁₃
- R₂ est un hydrogène
- Z₁ est un atome d'oxygène ou de soufre
- Ar est choisi parmi
   le radical IV dans lequel :
   - R₉ est un atome d'hydrogène
   ou le radical III dans lequel :
   - Z₂ est un radical C(R₇, R₈)
   - n=2

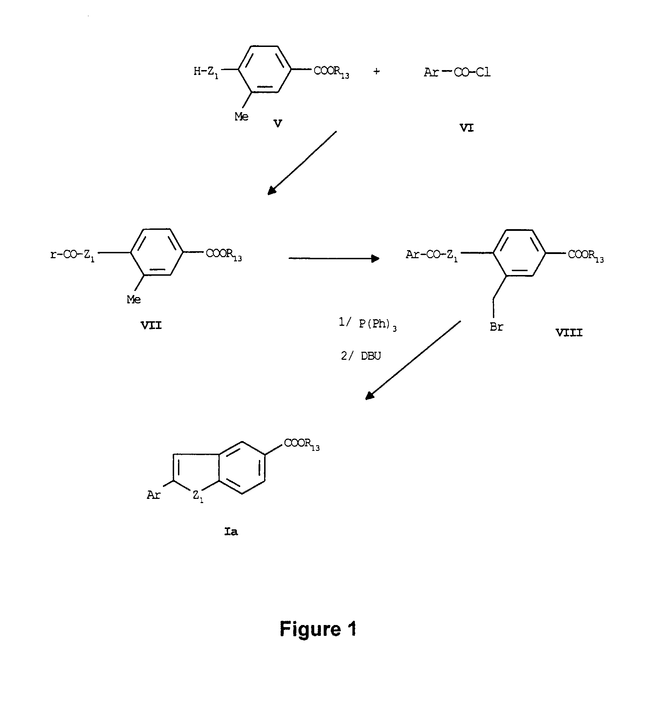

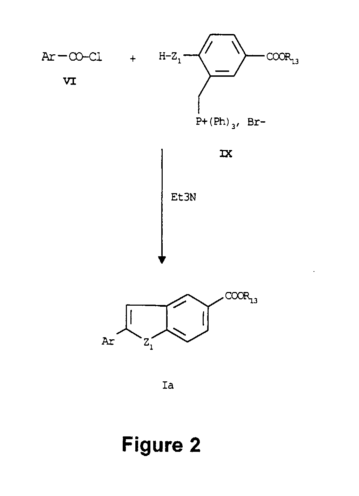

La présente invention a également pour objet les procédés de préparation des composés de formule (I), en particulier selon les schémas réactionnels donnés aux figures 1 et 2.

Ainsi les composés de formule générale (I) peuvent être obtenus (figure 1) par la méthode de type A.

Cette méthode consiste à faire réagir un dérivé (**V**) portant une fonction alcool, thiol ou amine sur un dérivé aromatique portant une fonction carboxylique activée (**VI**), Ar ayant la signification décrite dans la formule générale (I).

Le composé intermédiaire obtenu **VII** est alors soumis à une réction de bromation radicalaire pour conduire au dérivé **VIII**.

Aprés réaction en présence d'une triaryl phosphine ou d'un trialkyl phosphite, les dérivés résultants sont cyclisés en milieu basique. La base peut être un hydroxyde ou un carbonate de métal alcalin comme la lithine ou le carbonate de potassium, un hydrure de métal alcalin (hydrure de sodium), un alcoolate de métal alcalin (méthylate de sodium) ou une amine tertiaire (DBU, diazabicycloundécène) ou un amidure alcalin (di-isopropylamidure de lithium).

Les composés de formule générale (I) peuvent également être obtenus (figure 2) par la méthode de type B.

Dans cette méthode, les dérivés sont obtenus par réaction "one-pot" entre une forme activée de l'acide carboxylique aromatique **VI** sur un dérivé aromatique de formule **IX** portant une fonction alcool, thiol ou amino en ortho d'un groupe bromure de méthyltriphénylphosphonium. Cette réaction est effectuée en présence d'une amine tertiaire comme la triethylamine.

Les produits de formule générale (I) ainsi obtenus peuvent servir de produits de départ pour la fabrication d'autres composés de formule générale (I). Ces produits sont obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles sur le groupe R₁ comme indiqué ci-dessous:
- acide carboxylique: -> ester
- ester: -> acide carboxylique
- acide: -> chlorure d' acide
- chlorure d' acide: -> amide
- acide: -> amide
- acide: -> alcool
- alcool: -> aldéhyde
- amide: ->amine
- thiol: ->thioéther
- thioéther: -> sulfoxyde
- thioéther: -> sulfone
- acide sulfonique: -> ester sulfonique
- acide sulfonique: -> sulfonamide
- acide sulfinique: -> ester sulfinique

Les composés de formule générale (I) présentent une activité agoniste ou antagoniste vis à vis de l'expression d'un ou plusieurs marqueurs biologiques dans le test de différenciation des cellules (F9) de tératocarcinome embryonnaire de la souris (Skin Pharmacol. 3, p.256-267, 1990) et/ou sur la différenciation des kératinocytes humains in vitro (Skin Pharmacol. 3 p.70-85, 1990). Ces tests susmentionnés montrent les activités des composés dans les domaines de la différenciation et de la prolifération. Les activités peuvent aussi être mesurées dans des tests de transactivation cellulaire à l'aide de récepteurs recombinants RARs selon la méthode B.A.Bernard et al., Biochemical and Biophysical Research Communication, 1992, vol. 186, 977-983.

La présente invention a également pour objet à titre de médicament les composés de formule (I) tels que décrits ci-dessus.

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux, plus particulièrement les leucémies promyélocytaires.
12) Dans le traitement d'affections inflammatoires telles que l'arthrite.
13) Dans le traitement de toute affection d'origine virale au niveau cutané ou général.
14) Dans la prévention ou le traitement de l'alopécie.
15) Dans le traitement d'affections dermatologiques ou générales à composante immunologique.
16) Dans le traitement d'affections du système cardiovasculaire telles que l'artériosclérose.

Dans les domaines thérapeutiques mentionnés ci-dessus, les composés selon l'invention peuvent être avantageusement employés en combinaison avec d'autres rétinoïdes, avec des ligands des récepteurs RXR, avec des dérivés de la vitamine D, avec des corticostéroïdes ou des oestrogènes, en association avec des anti-oxydants, avec des α-hydroxy ou α-céto acides ou leurs dérivés, ou encore avec des bloqueurs de canaux potassiques.

Par ligand des récepteurs RXR, on entend, soit l'acide rétinoïque 9-cis, soit un analogue synthétique se liant sur ces RXR.

Par vitamines D ou leurs dérivés on entend par exemple les dérivés de la vitamine D₂ ou D₃ et en particulier la 1,25-dihydroxyvitamine D₃.

Par antiradicaux libres on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique, ascorbique, les dérivés d'acide salicylique, ainsi que leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc aussi pour objet une nouvelle composition médicamenteuse destinée notamment au traitement des affections susmentionées, caractérisée par le fait qu'elle comporte, dans un support pharmaceutiquement acceptable au moins un composé de formule (I), l'un de ses isomères optiques ou géométriques, ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.
Les composés selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I) telle que définie ci-dessus, l'un de ses isomères optiques ou géométriques ou un de ses sels, à une concentration de préférence comprise entre 0,001 et 5 % par rapport au poids total de la composition.

Les composés de formule (I) selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec d'autres rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques.

Les différents produits pris en association avec les composés de la présente invention étant tels que définis ci-dessus.

La présente invention vise donc également une composition cosmétique contenant, dans un support cosmétiquement acceptable, au moins un composé de formule I l'un de ses isomères optiques ou géométriques ou l'un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule I dans les compositions cosmétiques est comprise entre 0,001 et 3 % en poids.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphénylimidazolidine 2,4-dione) ; des agents anti-inflammatoires non stéroïdiens; des caroténoïdes et, notamment le b-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

On va maintenant donner, à titre d'illustration et sans aucun caractère limitatif, plusieurs exemples de préparation des composés actifs de formule I selon l'invention ainsi que des exemples de compositions les contenant.

### A. EXEMPLES DE COMPOSES

### 1) SELON LA VOIE DE SYNTHESE ILLUSTREE PAR LA FIGURE 1

### Exemple 1 : 2-(5,6,7,8-tétrahydro-5,5,8,8,-tetramethyl-2-naphthyl)-5-benzo(b)furanne carboxylate de méthyle

### a) Acide-3-méthyl-4-hydroybenzoïque

On mélange 32,4 g d'orthocrésol avec 375 ml (1,87 mole) de soude 5N, puis ajoute 25 g de b-cyclodextrine et 1,88 g de cuivre métallique en poudre. On ajoute en 10 minutes 65 ml (0,58 mmole) de tétrachorure de carbone et on chauffe à 80°C sous agitation pendant 16 heures. On refroidit le milieu réactionnel, on verse sur un mélange HCI 2N glacé et extrait à l'éther éthylique. La phase organique est lavée à l'eau, séchée sur sulfate de magnésium, filtrée et évaporée. Après purification sur silice en éluant avec de l'éther éthylique pur, on isole 40 g (88%) d'un solide rouge, utilisé directement pour l'étape 1b.

### b) 3-Méthyl-4-hydroxybenzoate de méthyle

Le composé obtenu dans l'exemple 1a est solubilisé dans 650 ml de méthanol et traité par 10 ml d'acide sulfurique concentré puis chauffé à reflux pendant 16 heures.
Après refroidissement, évaporation du méthanol et addition d'eau, le produit est extrait à l'éther éthylique, la phase organique est lavée à l'eau, séchée sur sulfate de magnésium et évaporée. Le produit est purifié par chromatographie sur silice en éluant par le mélange: dichlorométhane/éther éthylique (90:10), pour conduire à 33,1 g (76%) d'un solide rose. ¹H NMR (CDCl₃) d 2.27 (s, 3H), 3.89 (s, 3H), 6.83 (d, 1H, *J* = 8.3 Hz), 7.79 (d, 1H, *J* = 8.3 Hz), 7.84 (s, 1H).

### c) 3-Méthyl-4-(5,6,7,8-tetrahydro-5,5,8,8,-tetraméthyl-2-naphthoyloxy)-benzoate de méthyle

A 18,6 g (80 mmoles) d'acide 5,6,7,8-tetrahydro-5,5,8,8,-tetraméthyl-2-naphtalène carboxylique, on ajoute goutte à goutte 11,7 ml (160 mmoles) de chlorure de thionyle et chauffe le milieu à 80°C pendant 2h 30. Le milieu réactionnel est évaporé à sec et solubilisé dans 150 ml de THF anhydre. La solution ainsi obtenue est additionnée goutte à goutte sur une solution à 0°C contenant 13,3 g (80 mmoles) du phénol obtenu à l'exemple 1a et 12,3 ml (88 mmoles) de triéthylamine, dans 50ml de THF sec. Le milieu réactionnel est agité pendant 16 h à la température ambiante, puis on ajoute de l'eau et extrait à l'éther éthylique. La phase organique est lavée à l'eau, séchée sur sulfate de magnésium et évaporée. Le résidu obtenu est purifié sur silice, en éluant par un mélange dichloromethane/hexane (60:40) pour conduire, après évaporation des solvants, à 15,6 g (51%) du dérivé attendu, sous forme d'un produit solide blanc. ¹H NMR (CDCl₃) d 1.33 (s, 6H), 1.35 (s, 6H), 1.73 (s, 4H), 2.28 (s, 3H), 3.92 (s, 3H), 7.21 (d, 1H, *J* = 8.4 Hz), 7.46 (d, 1H, *J* = 8.3 Hz), 7.92 à 7.99 (m, 3H), 8.17 (d, 1 H, *J* = 1.8 Hz).

### d) 3-Bromométhyl-4-(5,6,7,8-tetrahydro-5,5,8,8,-tetraméthyl-2-naphthoyloxy)-benzoate de méthyle

A une solution de 6 g (17,35 mmoles) du dérivé obtenu dans l'exemple 1c, dans 70 ml de tétrachlorure de carbone, on ajoute 3,1g (17,35 mmoles) de N-bromosuccinimide, quelques cristaux de peroxyde de benzoyle et on chauffe le milieu réactionnel à 80°C pendant 8 heures. Après refroidissement et addition d'eau, le produit est extrait par du dichorométhane, la phase organique est lavée à l'eau, séchée sur sulfate de magnésium et évaporée. Après purification sur silice dans l'éluant: hexane/acétate d'éthyle (93:7), on isole 4,7g (65%) du dérivé attendu sous forme de produit solide blanc. ¹H NMR (CDCl₃) d 1.34 (br d, 12H), 1.73 (s, 4H), 3.92 (s, 3H), 4.49 (s, 2H), 7.40 (d, 1H, J = 8.5 Hz), 7.48 (d, 1H, *J* = 8.3 Hz), 8.00 (dd, 1H, *J* = 8.3 / 1.9 Hz), 8.07 (dd, 1H, *J* = 8.5 /1.9 Hz), 8.17 (d, 1H, *J* = 1.7 Hz), 8.26 (d, 1H, *J* = 1.7 Hz).

### e) 2-(5,6,7,8-tétrahydro-5,5,8,8,-tetraméthyl-2-naphtyl)-5-benzo(b)furanne carboxylate de méthyle

4,7 g (10,23 mmoles) du dérivé obtenu dans l'exemple 1d, en solution dans 40 ml de THF anhydre, sont traités par 3,1 g (11,7 mmoles) de triphényl phosphine et chauffés à 80°C pendant 4 heures. Après refroidissement à température ambiante, on ajoute goutte à goutte 1,76 ml (11,7 mmoles) de 1,8-diazabicyclo[5.4.0]undec-7-ene (DBU), agite une heure à 35°C, acidifie à pH 1 (HCI 2N), puis extrait par de l'éther éthylique. Après extraction par de l'éther éthylique, traitement habituel de la phase organique et évaporation, le produit est purifié sur silice dans le mélange: hexane/dichlorométhane, (60:40) pour conduire après évaporation, à 2,5 g (67%) du dérivé attendu sous forme d'un produit solide blanc. ¹H NMR (CDCl₃) d 1.31 (s, 6H), 1.37 (s, 6H), 1.72 (s, 4H), 3.94 (s, 3H), 7.01 (s, 1H), 7.39 (d, 1H, *J* = 8.3 Hz), 7.54 (d, 1H, *J* = 8.7 Hz), 7.60 (dd, 1H, *J* = 8.3 / 1.8 Hz), 7.81 (d, 1H, *J* = 1.8 Hz), 8.00 (dd, 1H, *J* = 8.6 / 1.7 Hz), 8.29 (d, 1H, *J* = 1.3 Hz).

### Exemple 2: Acide 2-(5,6,7,8-tetrahydro-5,5,8,8,-tetraméthyl-2-naphtyl)-5-benzo(b)furanne carboxylique

On place 2,5 g (6,9 mmoles) du composé obtenu dans l'exemple 1 dans 160 ml de méthanol et on les traite par 13,8 ml de soude 5N (69 mmoles). Le mélange réactionnel est chauffé à reflux pendant 2 heures. Après refroidissement, évaporation du méthanol et acidification, le résidu obtenu est extrait par de l'éther éthylique. La phase organique est alors lavée à l'eau, séchée et évaporée pour conduire à 2,4 g (100%) du dérivé attendu sous forme de solide blanc fondant à 273°C. ¹H NMR (DMSO d₆) d 1.27 (s, 6H), 1.33 (s, 6H), 1.67 (s, 4H), 7.46 (d, 1H, *J* = 8.3 Hz), 7.53 (s, 1H), 7.67 (dd, 1H, *J* = 8.3 /1.3 Hz), 7.73 (d, 1H, *J* = 8.6 Hz), 7.86 (d, 1H, *J* = 1.5 Hz), 7.93 (dd, 1H, *J* = 8.6 /1.6 Hz), 8.27 (d, 1H, *J* = 1.2 Hz), 12.93 (s, 1H).

### Exemple 3: 2-(5,6,7,8-tetrahydro-5,5,8,8,-tetraméthyl-2-naphtyl)-5-benzo(b)thiophène carboxylate de méthyle

### 3a) 3-Méthyl-4-(5,6,7,8-tetrahydro-5,5,8,8,-tetraméthyl-2-naphthoylthio)-benzoate de méthyle

A une solution contenant 23,2 g (100 mmoles) d'acide 5,6,7,8-tetrahydro-5,5,8,8,-tetraméthyl-2-naphtalène carboxylique dans 100 ml de dichlorométhane, on ajoute goutte à goutte 21,9 ml (110 mmoles) de dicyclohexylamine. Le milieu réactionnel est agité pendant 30 min à température ambiante, puis le dichlorométhane est évaporé et on reprend par 200 ml d'éther éthylique. Le précipité est séché puis on solubilise 6,8 g (16,5 mmoles) de ce sel dans 20 ml de dichlorométhane et on ajoute goutte à goutte 1,9 ml (26,3 mmoles) de chlorure de thionyle. Le milieu réactionnel est agité à température ambiante pendant 15 heures. Après filtration du chlorhydrate de dicyclohexylamine, le milieu réactionnel est évaporé, puis le chlorure d'acide obtenu est repris dans 30 ml de THF anhydre. On ajoute alors, goutte à goutte, une solution froide composée de 3 g (16,5 mmoles) de 3-méthyl-4-mercapto benzoate de méthyle, de 2,52 ml (18,1 mmoles) de triéthylamine en solution dans 10 ml de THF sec. Le milieu réactionnel est agité pendant une heure à température ambiante, puis on ajoute de l'eau et extrait le produit à l'éther éthylique. Après un traitement habituel de la phase organique et purification sur silice dans l'éluant hexane/ dichlorométhane (50:50), on isole 5,77 g (88%) du produit attendu, sous forme d'un solide blanc. ¹H NMR (CDCl₃) d 1.31 / 1.33 (d, 12H) , 1.72 (s, 4H), 3.94 (s, 3H), 7.43 (d, 1H, *J* = 8.3 Hz), 7.57 (d, 1H, *J* = 8.0 Hz), 7.81 (dd, 1H, *J* = 8.3 / 2.0 Hz), 7.90 (dd, 1H, *J* = 8.0 / 2.0 Hz), 7.97 (d, 1H, *J* = 1.9 Hz), 8.02 (d, 1H, *J* = 2.0 Hz).

### 3b) 3-Bromométhyl-4-(5,6,7,8-tetrahydro-5,5,8,8,-tetraméthyl-2-naphthoylthio)-benzoate de méthyle

On dissout 5,77g (14,5 mmoles) du dérivé obtenu dans l'exemple 3a, dans 70 ml de tetrachlorure de carbone, on ajoute 2,72 g (15,3 mmoles) de N-bromosuccinimide et chauffe le milieu réactionnel à reflux pendant 6 heures. Après refroidissement et addition d'eau, le produit est extrait par du dichlorométhane et la phase organique traitée conventionnellement. Après chromatographie sur silice dans le mélange hexane/ acétate d'éthyle, 90:10, on isole 5 g (72%) du produit attendu sous forme d'un solide blanc. ¹H NMR (CDCl₃) d 1.31 / 1.33 (d, 12H), 1.72 (s, 4H), 3.95 (s, 3H), 4.62 (s, 2H), 7.44 (d, 1H, *J* = 8.3 Hz), 7.63 (d, 1H, *J* = 8.1 Hz), 7.82 (dd, 1H, *J* = 8.3 /1.9 Hz), 7.98 (d, 1H, *J* = 1.9 Hz), 8.03 (dd, 1H, *J* = 8.1 / 1.8 Hz), 8.24 (d, 1H, *J*= 1.6 Hz).

### 3c) 2-(5,6,7,8-Tetrahydro-5,5,8,8,-tetraméthyl-2-naphtyl)-5-benzo(b)thiophène carboxylate de méthyle

5 g (10,5 mmoles) du composé obtenu à l'exemple 3b en solution dans 50 ml de THF anhydre sont traités par 3,2 g (12,1 mmoles) de triphénylphosphine et chauffés à reflux pendant 5 heures. Après refroidissement à température ambiante, on ajoute goutte à goutte 1,8 ml (12,1 mmoles) de DBU, agite une heure à 35°C, acidifie à pH 1 (HCI 2N), puis extrait par de l'éther éthylique. Après traitement habituel de la phase organique et purification sur silice dans l'éluant hexane/dichlorométhane (60:40), on obtient après évaporation, 2,65 g (66%) du dérivé attendu, sous forme d'un solide blanc. ¹H NMR (CDCl₃) d 1.31 (s, 6H), 1.35 (s, 6H), 1.72 (s, 4H), 3.96 (s, 3H), 7.37 (d, 1H, *J* = 8.2 Hz), 7.47 (dd, 1H, *J* = 8.2 / 1.9 Hz), 7.54 (s, 1H), 7.63 (d, 1H, *J* = 1.8 Hz), 7.84 (d, 1H, *J* = 8.5 Hz), 7.95 (dd, 1H, *J* = 8.5 / 1.5 Hz), 8.46 (d, 1H, *J* = 0.9 Hz).

### Exemple 4: Acide 2-(5,6,7,8-tetrahydro-5,5,8,8,-tetraméthyl-2-naphtyl)-5-benzo(b)thiophène carboxylique

Le composé obtenu dans l'exemple 3 (2,65 g ; 7,0 mmoles) en solution dans 150 ml de méthanol, est traité par 14 ml (70 mmoles) de soude 5N. Le mélange est chauffé à reflux pendant 2 heures. Après refroidissement, évaporation du méthanol et acidification, le produit est extrait par de l'éther éthylique. Après traitement de la phase organique, on recueille 2,36 g (92%) du dérivé attendu sous forme d'un produit solide blanc, fondant à 244°C. ¹H NMR (DMSO d₆) d 1.26 (s, 6H), 1.32 (s, 6H), 1.57 (s, 4H), 7.42 (d, 1H, *J* = 8.3 Hz), 7.50 (d, 1H, *J* = 8.3 Hz), 7.70 (s, 1H), 7.90 (d, 1H, *J* = 8.4 Hz), 7.98 (s, 1H), 8.07 (d, 1H, *J* = 8.4 Hz), 8.46 (s, 1H), 13.03 (s, 1H).

### Exemple 5 : 2-(1,2,3,4,4a,9b-Hexahydro-1,4a,9b-triméthyl-1,4-méthanodibenzofuran-8-yl)-benzo(b)thiophène-5-carboxylate de méthyle

### 5a) 3-Méthyl-4-(1,2,3,4,4a,9b-hexahydro-1,4a,9b-triméthyl-1,4-méthanodibenzofuran-8-carbonylthio)-benzoate de méthyle

8,5 g (18,7 mmoles) du sel de dicyclohexylamine de l'acide -1,2,3,4,4a,9b-hexahydro-1,4a,9b-triméthyl-1,4-méthanodibenzofuranne-8-carboxylique sont convertis en chlorure d'acide comme décrit dans la préparation de l'exemple 3a et sont mis en solution dans 40 ml de THF. On ajoute alors cette première solution goutte à goutte à une seconde, composée de 3,42 g (18,7 mmoles) de 2-méthyl-4-méthoxycarbonylthiophénol et 2,87 ml (20,6 mmoles) de triéthylamine dans 12 ml de THF. On laisse sous agitation 1h à température ambiante, ajoute de l'eau et extrait à l'éther éthylique. Après traitement habituel de la phase organique et chromatographie dans le mélange dichlorométhane/hexane (50:50), on isole 6,6 g (81%) du dérivé attendu sous forme d'un produit solide blanc. ¹H NMR (CDCl₃) d 0.86 à 1.68 (m, 6H), 1.21 (s, 3H), 1.27 (s, 3H), 1.39 (s, 3H), 2.28 (d, 1H, *J* = 4.1 Hz), 2.45 (s, 3H), 3.93 (s, 3H), 6.80 (d, 1H, *J* = 8.1 Hz), 7.57 (d, 1H, *J* = 8.1 Hz), 7.66 (d, 1H, *J* = 1.9 Hz), 7.90 (dd, 1H, *J* = 8.0 /1.4 Hz), 7.95 (dd, 1H, *J* = 8.5 / 2.0 Hz), 8.01 (d, 1H, *J* = 2.0 Hz).

### 5b) 3-Bromométhyl-4-(1,2,3,4,4a,9b-hexahydro-1,4a,9b-triméthyl-1,4-méthanodibenzofuran-8-carbonylthio)-benzoate de méthyle

Le dérivé obtenu dans l'exemple 5a (5,84 g ; 13,9 mmoles), placé dans 180 ml de tétrachlorure de carbone, est traité par 2,5 g (14,75 mmoles) de N-bromosuccinimide. Le mélange réactionnel est chauffé à 70°C pendant 24 heures pour conduire après le même traitement que dans l'exemple 1d à 2,2 g (32%) du dérivé attendu sous forme d'un produit solide blanc. ¹H NMR (CDCl₃) d 0.86 à 1.68 (m, 6H), 1.21 (s, 3H), 1.27 (s, 3H), 1.40 (s, 3H), 2.28 (d, 1H, *J* = 4.0 Hz), 3.95 (s, 3H), 4.64 (s, 2H), 6.81 (d, 1H, *J* = 8.5 Hz), 7.63 (d, 1H, *J* = 8.1 Hz), 7.66 (d, 1H, *J* = 1.9 Hz), 7.97 (dd, 1H, *J* = 8.5 / 1.9 Hz), 8.02 (dd, 1H, *J* = 8.1 /1.8 Hz), 8.23 (d, 1H, *J* = 1.6 Hz).

### 5c) 2-(1,2,3,4,4a,9b-Hexahydro-1,4a,9b-triméthyl-1,4-méthanodibenzofuran-8-yl)-benzo(b)thiophène-5-carboxylate de méthyle.

Le composé obtenu dans l'exemple 5b (2,2 g, 4,27 mmoles), en solution dans 20 ml de THF, est traité par 1,3 g (4,9 mmoles) de triphénylphosphine, puis par 0,73 ml (4,9 mmoles) de DBU, dans les conditions décrites de l'exemple 1. On isole, après le même traitement, suivi d'une chromatographie dans le mélange dichlorométhane/hexane, (40:60 ), 0,64 g (36 %) du dérivé attendu, sous forme d'un produit solide blanc. ¹H NMR (CDCl₃) d 0.85 à 1.68 (m, 6H), 1.24 (s, 3H), 1.30 (s, 3H), 1.39 (s, 3H), 2.26 (d, 1H, *J* = 4.1 Hz), 3.96 (s, 3H), 6.79 (d, 1H, *J* = 8.3 Hz), 7.32 (d, 1H, *J* = 1.7 Hz), 7.44 (s, 1H), 7.50 (dd, 1H, *J* = 8.3 /1.8 Hz), 7.82 (d, 1H, *J* = 8.4 Hz), 7.93 (dd, 1H, *J* = 8.5 / 1.4 Hz), 8.43 (d, 1H, *J* = 1.7 Hz).

### Exemple 6 : Acide 2-(1,2,3,4,4a,9b-hexahydro-1,4a,9b-triméthyl-1,4-méthanodibenzofuran-8-yl)-benzo(b)thiophène-5-carboxylique

Le composé obtenu dans l'exemple 5 (0,64 g ; 1,53 mmole), est placé dans 35 ml de méthanol et 100 ml de THF, puis on ajoute 7,6 ml de soude 2N et chauffe à 40°C pendant 2 heures. Après le même traitement que pour isoler le composé de l'exemple 1, suivi d'un empatage dans l'hexane, on obtient 0,54 g (87%) du dérivé attendu, sous forme d'un produit solide blanc fondant à 256°C. ¹H NMR (DMSO d₆) d 0.83 à 0.92 (m, 2H), 1.05 à 1.15 (m, 2H), 1.22 (s, 3H), 1.28 (s, 3H), 1.35 (s, 3H), 1.53 (m, 2H), 1.72 (d, 1H, *J* = 10.2 Hz), 2.20 (d, 1H, *J* = 3.2 Hz), 6.83 (d, 1H, *J* = 8.9 Hz), 7.52 à 7.54 (d + s, 2H), 7.84 à 7.88 (d + s, 2H), 8.04 (d, 1H, *J*= 8.4 Hz), 8.39 (s, 1H), 12.98 (s, 1H).

### Exemple 7: Acide 2-(5,6,7,8-tetrahydro-5,5,8,8,-tetraméthyl-3-méthoxy-2-naphtyl)-5-benzo(b)furanne carboxylique

### 7a) 2-Méthoxy-3-acétyl-5,6,7,8-tetrahydro-5,5,8,8,-tetraméthylnaphtyl naphtalène

40 g (162,3 mmoles) de 2-hydroxy-3-acétyl-5,6,7,8-tetrahydro-5,5,8,8,-tetraméthyl naphtylnaphtalène sont dissous dans 250 ml de DMF et sont ensuite refroidis à 0°C. On ajoute alors lentement 5,12 g (170,5 mmoles) d' hydrure de sodium, puis ajoute goutte à goutte 24,2 ml (170,5 mmoles) d'iodure de méthyle. Le mélange réactionnel est laissé sous agitation une nuit à température ambiante, puis versé dans de l'eau glacée. On extrait par de l'éther éthylique puis, après un traitement habituel suivi d'une chromatographie dans la mélange dichlorométhane/hexane (40:60), on isole 36,0 g (85%) d'un produit cristallisé blanc cassé. ¹H NMR (CDCl₃) d 1.27 (s, 6H), 1.30 (s, 6H), 1.68 (s, 4H), 2.59 (s, 3H), 3.89 (s, 3H), 6.85 (s, 1H), 7.73 (s, 1H).

### 7b) Acide 2-Méthoxy-5,6,7,8-tetrahydro-5,5,8,8, tetraméthyl naphtalène-3-carboxylique

A une solution de 440 ml de soude 5 N, refroidie à 0°C, on ajoute 91 g (570 mmoles ) de brome, puis une solution de 35,9 g (138 mmoles) du composé obtenu dans l'exemple 7a en solution dans 275 ml de dioxanne. Le milieu réactionnel est agité à température ambiante pendant 4 heures, puis neutralisé avec HCI 5N et extrait à l'éther éthylique. On lave la phase organique à l'eau, au thiosulfate de sodium, la rince jusqu'à pH neutre, sèche, filtre et évapore les solvants. On purifie sur silice avec l'éluant éther éthylique/hexane (40: 60). On obtient 27,7 g (77%) du dérivé attendu, sous forme d'un solide blanc rosé. ¹H NMR (CDCl₃) d 1.28 (s, 6H), 1.31 (s, 6H), 1.69 (s, 4H), 4.06 (s, 3H), 6.93 (s, 1H), 8.12 (s, 1H).

### 7c) 2-(5,6,7,8-Tetrahydro-5,5,8,8,-tetraméthyl-3-méthoxy-2-naphtyl)-5-benzo(b)furanne carboxylate de méthyle

5,25 g (20 mmoles) de l'acide obtenu dans l'exemple 7b sont convertis en chlorure d'acide, comme décrit dans l'exemple 1c, et sont dissous dans 90 ml de toluène. On ajoute alors cette solution goutte à goutte à une solution contenant 10,65 g (21 mmoles) de bromure de 2-hydroxy-4-méthoxycarbonylbenzyltriphényl phosphonium (obtenu d'après la méthode décrite dans le brevet EP 732 328) et et 7,6 ml (54,4 mmoles) de triéthylamine dans 150 ml de toluène. On chauffe à reflux le mélange réactionnel pendant 15 minutes. On refroidit, reprend par de l'éther éthylique, acidifie par de l' HCI 2N et extrait à l'éther éthylique. Après le traitement habituel de la phase organique et purification sur silice dans l'éluant dichlorométhane/hexane (60:40), on obtient 2,95 g (38%) d'un produit solide blanc. ¹H NMR (CDCl₃) d 1.33 (s, 6H), 1.36 (s, 6H), 1.72 (s, 4H), 3.94 (s, 3H), 3.98 (s, 3H), 6.91 (s, 1H), 7.32 (s, 1H), 7.54 (d, 1H, *J*= 8.6 Hz), 7.96 (s, 1H), 7.99 (dd, 1H, *J* = 8.6 /1.8 Hz), 8.30 (d, 1H, *J* = 1.2 Hz).

### Exemple 8: Acide 2-(5,6,7,8-tetrahydro-5,5,8,8,-tetraméthyl-3-méthoxy-2-naphtyl)-5-benzo(b)furanne carboxylique

2,9 g (7,39 mmoles) du composé obtenu dans l'exemple 7 en solution dans 160 ml de méthanol, sont traités par 15 ml de soude 5N dans les conditions décrites pour la synthèse de l'exemple 2. On isole après le même traitement, suivi d'une recristallisation dans un mélange éther éthylique/hexane (30:70), 2,43 g (87%) d'un produit solide blanc fondant à 280°C. ¹H NMR (CDCl₃) d 1.33 (s, 6H), 1.36 (s, 6H), 1.72 (s, 4H), 3.99 (s, 3H), 6.91 (s, 1H), 7.32 (s, 1H), 7.54 (d, 1H, *J* = 8.6 Hz), 7.95 (s, 1H), 8.01 (dd, 1H, *J* = 8.6 /1.7 Hz), 8.32 (d, 1H, *J* = 1.3 Hz).

### Exemple 9: 2-(5,6,7,8-Tetrahydro-5,5,8,8,-tetraméthyl-3-n-propyloxy-2-naphtyl)-5-benzo(b)furanne carboxylate de méthyle

### 9a) 2-n-Propyloxy-3-acétyl-5,6,7,8-tetrahydro-5,5,8,8,-tetraméthylnaphtyl naphtalène

On traite 35,6 g (0,14 mole) de 2-hydroxy-3-acétyl-5,6,7,8-tetrahydro-5,5,8,8,-tetraméthynaphtylnaphtalène en solution dans 300 ml de DMF, par 4,5 g d'hydrure de sodium, puis par 18,7 g de bromure de n-propyle, dans les conditions décrites dans l'exemple 7a. On isole, après le même traitement et une chromatographie sur silice dans l'éluant éther éthylique/hexane (5:95), 27,3 g (65%) du dérivé attendu sous forme d'un solide blanc. ¹H NMR (CDCl₃) d 1.08 (t, 3H), 1.27 (s, 6H), 1.29 (s, 6H), 1.67 (s, 4H), 1.87 (m, 2H), 2.62 (s, 3H), 4.01 (t, 2H), 6.83 (s, 1H), 7.75 (s, 1H).

### 9b) Acide 2-n-propyloxy-5,6,7,8-tetrahydro-5,5,8,8,-tetraméthyl naphtalène-3-carboxylique

On traite 27,3 g du composé obtenu dans l'exemple 9a, en solution dans 190 ml de dioxanne, par une solution d'hypobromite de sodium constituée de 300 ml de soude 5 N, placée à 0°C et 62,4 g de brome dans les conditions décrites dans l'exemple 7b. On isole après le même traitement, 24,1 g (88%) du composé attendu, sous forme d'un solide blanc. ¹H NMR (CDCl₃) d 1.10 (t, 3H), 1.28 (s, 6H), 1.30 (s, 6H), 1.69 (s, 4H), 1.94 (m, 2H), 4.20 (t, 2H), 6.91 (s, 1H), 8.12 (s, 1H), 11.06 (br s, 1H).

### 9c) 2-(5,6,7,8-Tetrahydro-5,5,8,8,-tetraméthyl-3-n-propyloxy-2-naphtyl)-5-benzo(b)furanne carboxylate de méthyle

5,8 g (20 mmoles) de l'acide obtenu dans l'exemple 9b, sont convertis en chlorure d'acide, comme décrit dans l'exemple 1c, puis sont traités par 10,65 g (21 mmoles) de bromure de 2-hydroxy-5-méthoxycarbonylbenzyltriphényl phosphonium, et 7,6 ml (54,4 mmoles) de triéthylamine dans les conditions décrites dans l'exemple 5 (synthèse de type b). Après le même traitement, suivi d'une chromatographie sur silice dans l'éluant: dichlorométhane/hexane (40:60), on obtient 3,1 g (37%) d'un produit solide blanc. ¹H NMR (CDCl₃) d 1.15 (t, 3H), 1.32 (s, 6H), 1.36 (s, 6H), 1.71 (s, 4H), 1.97 (m, 2H), 3.94 (s, 3H), 4.09 (t, 2H), 6.89 (s, 1H), 7.36 (s, 1H), 7.54 (d, 1H, J = 8.6 Hz), 7.96 (s, 1H), 7.99 (dd, 1H, J = 8.6 / 1.6 Hz), 8.32 (d, 1H, *J* = 1.4 Hz).

### Exemple 10: Acide 2-(5,6,7,8-tetrahydro-5,5,8,8,-tetraméthyl-3-n-propyloxy-2-naphtyl)-5-benzo(b)furanne carboxylique

3,05 g (7,25 mmoles) du composé obtenu dans l'exemple 9, en solution dans 160 ml de méthanol, sont traités par 14,5 ml de soude 5N dans les conditions décrites dans la synthèse de l'exemple 2. On isole, après le même traitement, suivi d'une recristallisation dans un mélange éther éthylique/hexane (30:70), 2,7 g (92%) d'un produit solide blanc fondant à 256°C. ¹H NMR (CDCl₃) d 1.15 (t, 3H), 1.32 (s, 6H), 1.36 (s, 6H), 1.72 (s, 4H), 1.98 (m, 2H), 4.10 (t, 2H), 6.89 (s, 1H), 7.35 (s, 1H), 7.54 (d, 1H, *J* = 8.6 Hz), 7.96 (s, 1H), 8.01 (dd, 1H, *J* = 8.6 / 1.7 Hz), 8.32 (d, 1H, *J* = 1.2 Hz).

### Exemple 11: 2-(5,6,7,8-Tetrahydro-5,5,8,8,-tetraméthyl-3- n-heptyloxy-2-naphtyl)-5-benzo(b)furanne carboxylate de méthyle

### 11a) 2-n-Heptyloxy-3-acétyl-5,6,7,8-tetrahydro-5,5,8,8,-tetraméthylnaphtalène

On traite 40 g (0,16 mole) de 2-hydroxy-3-acétyl-5,6,7,8-tetrahydro-5,5,8,8,-tetraméthynaphtylnaphtalène en solution dans 350 ml de DMF, par 5,1 g d'hydrure de sodium puis par 26,8 ml de bromure de n-heptyle dans les conditions décrites dans l'exemple 7a. On isole, après le même traitement suivi d'une chromatographie sur silice dans l'éluant dichlorométhane/hexane (40:60), 42,9 g (77%) du dérivé attendu sous forme d'une huile jaune. ¹H NMR (CDCl₃) d 0.82 à 0.92 (m, 5H), 1.27 (s, 6H), 1.29 (s, 6H), 1.30 à 1.51 (m, 6H), 1.67 (s, 4H), 1.84 (m, 2H), 2.62 (s, 3H), 4.03 (t, 2H), 6.82 (s, 1H), 7.75 (s, 1H).

### 11b) Acide 2-n-heptyloxy-5,6,7,8-tetrahydro-5,5,8,8,-tetraméthylnaphtalène-3-carboxylique

On traite 42,7 g du composé obtenu dans l'exemple 11a en solution dans 250 ml de dioxanne, par une solution d'hypobromite de sodium constituée de 397 ml de soude 5 N, refroidie à 0°C, et 81,8 g de brome dans les conditions décrites dans l'exemple 7b. On isole après le même traitement, suivi d'une chromatographie sur silice, 39,9 g (93%) du dérivé attendu sous forme d'un produit solide blanc. ¹H NMR (CDCl₃) d 0.90 (t, 3H), 1.28 (s, 6H), 1.30 (s, 6H), 1.30 à 1.49 (m, 8H), 1.69 (s, 4H), 1.91 (m, 2H), 4.22 (t, 2H), 6.91 (s, 1H), 8.12 (s, 1H).

### 11c) 2-(5,6,7,8-Tetrahydro-5,5,8,8,-tetraméthyl-3-n-heptyloxy-2-naphtyl)-5-benzo(b)furanne carboxylate de méthyle

6,9 g (20 mmoles) de l'acide obtenu dans l'exemple 11b, sont convertis en chlorure d'acide, comme décrit dans l'exemple 1c, puis sont traités par 10,65 g (21 mmoles) de bromure de 2-hydroxy-5-méthoxycarbonylbenzyltriphénylphosphonium et 7,6 ml (54,4 mmoles) de triéthylamine dans les conditions décrites dans l'exemple 7c. On obtient, après le même traitement, suivi d'une chromatographie sur silice dans l'éluant: dichlorométhane/heptane (35:65), 3,8g (40%) d'un produit solide blanc. ¹H NMR (CDCl₃) d 0.92 (t, 3H), 1.32 (s, 6H), 1.36 (s, 6H), 1.25 à 1.59 (m, 8H), 1.71 (s, 4H), 1.96 (m, 2H), 3.94 (s, 3H), 4.11 (t, 2H), 6.88 (s, 1H), 7.35 (s, 1H), 7.54 (d, 1H, J = 8.6 Hz), 7.96 (s, 1H), 7.99 (dd, 1H, *J* = 8.6 / 1.7 Hz), 8.31 (d, 1H, *J* = 1.2 Hz).

### Exemple 12: Acide 2-(5,6,7,8-tetrahydro-5,5,8,8,-tetraméthyl-3-n-heptyloxy-2-naphtyl)-5-benzo(b)furanne carboxylique

3,7 g (7,8 mmoles) du composé obtenu dans l'exemple 11 en solution dans 160 ml de méthanol, sont traités par 15,7 ml de soude 5N dans les conditions décrites pour la synthèse de l'exemple 2. On isole, après le même traitement suivi d'une recristallisation dans un mélange éther éthylique/hexane 3,5 g (98%) d'un produit solide blanc fondant à 200°C. ¹H NMR (CDCl₃) d 0.93 (t, 3H), 1.33 (s, 6H), 1.37 (s, 6H), 1.34 à 1.61 (m, 8H), 1.72 (s, 4H), 1.96 (m, 2H), 4.12 (t, 2H), 6.89 (s, 1H), 7.38 (s, 1H), 7.59 (d, 1H, J= 8.6 Hz), 7.97 (s, 1H), 8.08 (dd, 1H, *J* = 8.6 / 1.7 Hz), 8.41 (d, 1H, *J* = 1.3 Hz).

### Exemple 13: Acide 2-(5,6,7,8-tétrahydro-5,5,8,8-tétraméthyl-2-naphtyl)-5-indolecarboxylique

### 13a) 3-Méthyl-4-(5,6,7,8-tétrahydro-5,5,8,8-tétraméthyl-2-naphtylcarbonyl amino)-benzoate de méthyle

L'acide 5,6,7,8-tétrahydro-5,5,8,8-tétraméthyl-2-naphtalène carboxylique (37,7g ; 162,6mmoles) est converti en chlorure d'acide comme indiqué dans l'exemple 3a.Après le même traitement, ce chlorure d'acide est ajouté à une solution refroidie à 0°C contenant 25 g (157,3 mmoles) de 3-méthyl-5 hydroxybenzoate de méthyle et 16,2ml de triéthylamine en solution dans 500 ml de THF. Le milieu réactionnel est agité pendant 16 heures à température ambiante et traité comme indiqué à l'exemple 1c, pour conduire à 55,12 g (86%) du dérivé attendu, sous forme d'un produit solide blanc, fondant à 177-178°C. ¹H NMR (CDCl₃) d 1.31 (s, 6H), 1,34 (s, 6H); 1,72 (s, 4H), 2,38(s, 3H), 3,90(s, 3H), 7,42 (d, J= 8, 1H); 7,53 (dd, J = 8 / 2 Hz, 1H), 7,81 (br, NH), 7,89-7,96 (m, 3H); 8,32 (d, J= 8Hz, 1H).

### 13b) 3-Méthyl-4-[N-tert-Butoxycarbonyl-(5,6,7,8-tétrahydro-5,5,8,8-tétraméthyl-2-naphtamido)]-benzoate de méthyle

L'amide obtenu à l'exemple 13a(48g, 126,5 mmoles) est dissout dans 200ml de DMF puis est traité par 7,6g d'hydrure de sodium (à 80%) introduit en peptites quantités. Après cessation du dégagement d'hydrogène, on ajoute goutte à goutte 55,2g(2eq) de ditertbutyldicarbonate) en solution dans 50ml de DMF. Le milieu réactionnel est laissé sous agitation à température ambiante pendant 16h. La réaction est versée dans l'eau, extraite à l'ether, lavée et rincée pour conduire à 59g (97%) du dérivé attendu sous forme d'une huile rose.
¹H NMR (CDCl₃) d :1,22 (s, 9H), 1,28 (s, 12H), 1,70 (s, 4H); 2,35 (s, 3H), 3,91 (s, 3H), 7,25 (d, 2Hz, 1H), 7,36 (d, 8Hz, 1H); 7,47 (dd, 8 / 2 Hz, 1H); 7,65 (d, 2Hz, 1H), 7,92 (dd, 8 / 2 Hz, 1H), 7,98 (s, 1H).

### 13c) 3-Bromométhyl-4-[N-tert-Butoxycarbonyl-(5,6,7,8-tétrahydro-5,5,8,8-tétraméthyl-2-naphtamido)]-benzoate de méthyle

Le dérivé obtenu à l'exemple 13b (24,5g, 51 mmoles) est mis en solution dans du tétrachlorure de carbone est traité par 9g (50,6 mmoles) de N-bromosuccinimide , 54g (51 mmoles) de carbonate de sodium et 0,37g (1,5 mmoles) de péroxyde de benzoyle. La réaction est irradiée par une lampe de 1000W durant 30 min. Après évaporation, le résidu est chromatographié sur silice et élué dans le mélange CH₂Cl₂ / heptane (20:80) pour conduire à 10,5g (36%) d'un produit amorphe.
¹H NMR (CDCl₃) δ :1,24 (s, 9H); 1,30 (s, 6H); 1,32 (s, 6H); 1,71 (s, 4H) ; 3,94 (s, 3H), 4,49 (s, 2H) ; 7,33 (d, J = 8Hz, 1H) ; 7,40 (d, J = 8Hz), 1H); 7,53 (dd, J = 8 / 2 Hz, 1H) ; 7,78 (s, 1H) ; 8,09 (dd, J = 2 / 8, 1H) ; 8,20 (s, 1H).

### 13d) 1-tert-Butyloxycarbonyl-2-(5,6,7,8-tétrahydro-5,5,8,8-tétraméthyl-2-naphtamido)-5-indole carboxylate de méthyle

Le dérivé bromométhyle obtenu à l'exemple 13c (9g, 16,1 mmoles) est dissout dans du THF anhydre (100ml). On ajoute à cette solution 5g (19,3 mmoles de triphénylphosphine. On chauffe la réaction à reflux pendant 4 h. On ajoute alors 2,94g (19,3 mmoles) de diazabicyclo-undécène à température ambiante et laisse agiter à cette température pendant une heure.
Le milieu réactionnel est versé dans l'eau, extrait par du dichlorométhane, lavé, séché et évaporé. Ce résidu est chromatographié sur silice dans l'éluant CH2Cl2 / heptane, 60 :40 pour conduire à 4,8g (65%) du résidu attendu sous forme d'un solide blanc fondant à 125-130°C.
¹H NMR (CDCl₃) δ:1,26 (s, 9H), 1,31 (s, 12H); 1,72 (s, 4H); 3,95(s, 3H); 6,59(s, 1H) ; 7,13(dd, J = 1,5 / 8 Hz, 1H); 7,3 (m, 2H), 8,01 (dd, J = 1,7 / 9 Hz, 1H), 8,23 (d, J = 11 Hz, 1H) ; 8,28 (d, 1,7Hz, 1H).

### 13e) Acide 2-(5,6,7,8-tétrahydro-5,5,8,8-tétraméthyl-2-naphtyl)-5-indole carboxylique

Une solution de 1,85g(4mmoles) de l'ester méthylique sont traités par 1,6g(40mmoles) de soude dans 25ml de méthanol. Le milieu réactionnel est chauffé 13h à reflux puis est laissé 15h à température ambiante. Après évaporation du méthanol, le résidu est repris dans l'eau puis est acidifié. On extrait par de l'éther éthylique, lave à l'eau sèche et évapore pour obtenir après trituration dans l'heptane, 1,34g (96%) d'une poudre blanche fondant à 265°C.
¹H NMR (CDCl₃) δ : 1,27(s, 6H); 1,34(s, 6H) ; 1,67 (s, 4H) ; 7,0(s, 1H) ; 7,4 (d, J = 8Hz, 1H) ; 7,45 (d, J = 8Hz, 1H) ; 7,62 (dd, J = 1,5 / 8,2Hz, 1H) ; 7,73 ( dd, J = 1,5 / 8,5Hz, 1H) ; 7,82 ( s, 1H) ; 8,2(s, 1H) ; 11,82(s, 1H).

### B. EXEMPLES DE FORMULATION

### 1) VOIE ORALE

(a) On prépare la composition suivante sous la forme d'un comprimé de 0,8 a

| | |
|---|---|
| Composé de l'exemple 1 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Pour le traitement de l'acné, on administre à un individu adulte 1 ampoule par jour pendant 3 mois selon la gravité du cas traité.
(c) On prépare la formulation suivante destinée à être conditionnée en gélules :

| | |
|---|---|
| Composé de l'exemple 3 | 0,025 g |
| Amidon de maïs | 0,060 g |
| Lactose q.s.p. | 0,300 g |

Les gélules utilisées sont constituées de gélatine, d'oxyde de titane et d'un conservateur.

Dans le traitement du psoriasis, on administre à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 4 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours.
(b) On prépare un gel en réalisant la formulation suivante :

| | |
|---|---|
| Composé de l'exemple 5 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 6 | 0,030 g |
| Propylène glycol | 5,000 g |
| Butylhydroxytoluène | 0,100 g |
| Ethanol (à 95°) q.s.p. | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 7 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p. | 100,000 g |

Cette composition est appliquée quotidiennement, elle permet de lutter contre le vieillissement photoinduit.
(e) On prépare la crème Huile dans l'Eau non ionique suivante :

| | |
|---|---|
| Composé de l'exemple 8 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 Jours.
(f) On prépare un gel topique en procédant au mélange des ingrédients suivants :

| | |
|---|---|
| Composé de l'exemple 9 | 0,050 g |
| Ethanol | 43,000 g |
| a -tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 10 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 12 | 0,050 g |
| Acide rétinoïque | 0,010 g |
| Mélange de stéarates de glycérol et de polyéthylène glycol (75 moles) vendu sous le nom de "Gelot 64" ..par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 13 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p. | 100,000 g |

Cette crème est appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 11 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000g |
| Mélange de triglycérides caprylique-caprique vendu sous la dénomination de "Miglyol 812" . par la société "DYNAMIT NOBEL" | 4,000 g |
| Eau q.s.p. | 100,000 g |

Cette crème est appliquée 1 fois par jour, elle aide à lutter contre le vieillissement qu'il soit photoinduit ou chronologique.

## Revendications

1. Composés, caractérisés par le fait qu'ils répondent à la formule générale (I) suivante : 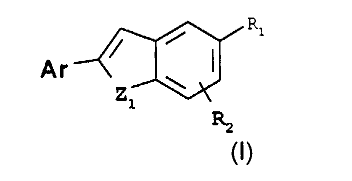 dans laquelle :
Ar représente un radical choisi parmi les radicaux de formules (II)-(IV) suivantes: 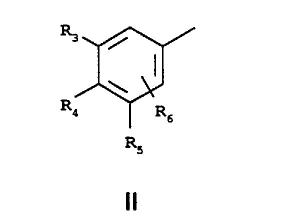 R₃, R₄, R₅, et R₆ ayant les significations données ci-après, 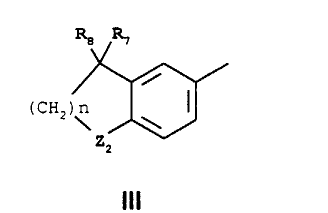 n, Z₂, R₇ et R₈ ayant les significations données ci-après, 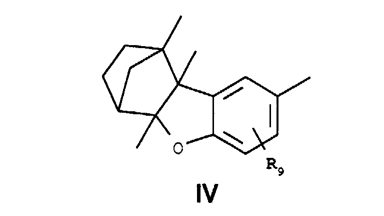 R₉ ayant la signification donnée ci-après,
Z₁ représente un atome d'oxygène, de soufre ou le radical NR',
R' ayant la signification donnée ci-après,
Z₂ représente C(R₇R₈), O, NR', S, SO, ou SO₂,
R₇, R₈, R' ayant les significations données ci-après,
R₁ représente
(i) un radical -CH₃,
(ii) un radical -(CH₂)ₘ-O-R_{10,}
(iii) un radical -CH₂-O-CO-R₁₁
(iv) un radical -(CH₂)ₓ-CO-R₁₂
(v) un radical -(CH₂)ₓ-CO-OR₁₃
R₁₀, R₁₁, R₁₂ et R₁₃, m, x et t ayant les significations données ci-après,
R₂ représente un atome d'hydrogène, un atome d'halogène, un radical alkoxy linéaire ou ramifié de 1 à 20 atomes de carbone, ou un radical -O-CH₂-O-CH₂-CH₂-O-CH₃,
R₃ et R₅ identiques ou différents représentent un atome d'hydrogène, un radical alkyle, ou un radical cycloalkyle,
avec les conditions suivantes :
- R₃ et R₅ ne représentent pas simultanément un atome d'hydrogène,
- lorsque R₃ ou R₅ représente un radical adamantyl alors Z₁ est différent d'un atome d'oxygène,
R₄ représente un radical -O-CH₂-O-CH₂-CH₂-O-CH₃, un radical -(Y)_{q}-(CH₂)ₛ-R₁₄, un radical -(CH₂)_{z}-Y-(CH₂)ₛ-R₁₄, ou un radical -CH=CH-(CH₂)_{w}-R₁₄,
Y et R₁₄ ayant les significations données ci-après,
q, z, s, w, identiques ou différents ayant les significations données ci-après,
R₈ représente un atome d'hydrogène, un atome d'halogène, un radical alkoxy linéaire ou ramifié de 1 à 20 atomes de carbone ou un radical -O-CH₂-O-CH₂-CH₂-O-CH₃,
R₇ et R₈ identiques ou différents représentent un radical alkyle inférieur,
R₉ représente un atome d'hydrogène, un atome d'halogène, un radical alkoxy linéaire ou ramifié de 1 à 20 atomes de carbone, ou un radical -O-CH₂-O-CH₂-CH₂-O-CH₃,
R₁₀ représente un hydrogène ou un radical alkyle inférieur,
R₁₁ représente un radical alkyle inférieur,
R₁₂ représente un atome d'hydrogène, un radical alkyle inférieur ou un radical -N(R",R"'),
R" et R"' ayant les significations données ci-après,
R₁₃ représente un atome d'hydrogène, un radical alkyle linéaire ou ramifié de 1 à 20 atomes de carbone, un radical alkényle, un radical mono- ou polyhydroxyalkyle,
R₁₄ représente un radical choisi parmi :
(i) un atome d'hydrogène,
(ii) un radical alkyle inférieur,
(iii) un radical alkényle,
(iv) un radical alkynyle,
(v) un radical cycloaliphatique ayant de 3 à 6 atomes de carbone,
(vi) un radical mono ou polyhydroxyalkyle, les groupements hydroxyles pouvant optionnellement être protégés sous forme de méthoxy, acétoxy ou acétonide,
(vii) un radical CO-R₁₂,
(viii) un radical COO-R₁₃,
(ix) un radical hydroxyle, un radical O-R₁₅ ou O-CO-R₁₅, à la condition que R₄ représente le radical -(Y)_{q}-(CH₂)ₛ-R₁₄ avec q est égal à 0,
Y, et R₁₅ ayant les significations données ci-après,
q et s ayant les significations données ci-après,
R₁₅ représente un radical alkyle inférieur,
R' représente un atome d'hydrogène, un radical alkyle inférieur ou un groupement protecteur de la fonction amine,
R" et R"' identiques ou différents, représentent un atome d'hydrogène, un radical alkyle inférieur, un radical mono ou polyhydroxyalkyle, ou encore, R" et R"' pris ensemble, forment un hétérocycle,
Y représente S, O ou S(O)t,
t ayant la signification donnée ci-après,
m représente un nombre entier pouvant prendre une valeur allant de 0 à 2,
n représente un nombre entier pouvant prendre la valeur 1 ou 2,
q représente un nombre entier pouvant prendre la valeur 0 ou 1,
s représente un nombre entier pouvant prendre une valeur entière allant de 0 à 12,
t représente un nombre entier pouvant prendre une valeur allant de 0 à 3,
w représente un nombre entier pouvant prendre une valeur entière allant de 0 à 10,
x représente un nombre entier pouvant prendre une valeur allant de 0 à 2,
z représente un nombre entier pouvant prendre la valeur 1, 2 ou 3, et les isomères optiques et géométriques desdits composés de formule (I) ainsi que leurs sels .

2. Composés selon la revendication 1, caractérisés par le fait qu'ils se présentent sous forme de sels d'un métal alcalin ou alcalino-terreux, de zinc, d'une amine organique, d'un acide minéral ou organique.

3. Composés selon l'une des revendications 1 ou 2, caractérisés par le fait que les radicaux alkyles inférieurs sont choisis parmi les radicaux méthyle, éthyle, isopropyle, n-butyle ou tertiobutyle.

4. Composés selon l'une des revendications précédentes, caractérisés en ce que le radical cycloalkyle éventuellement substitué par un ou des atomes d'halogène ou un ou des radicaux hydroxyles correspond à un radical adamantyle ou un radical 1-méthylcyclohexyle.

5. Composés selon l'une des revendications précédentes, caractérisés en ce que les radicaux monohydroxyalkyles sont choisis parmi les radicaux 2-hydroxyéthyle, 2-hydroxypropyle ou 3-hydroxypropyle.

6. Composés selon l'une des revendications précédentes, caractérisés en ce que les radicaux polyhydroxyalkyles sont choisis parmi les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou par un mono ou polyhydroxyalkyle.

8. Composés selon la revendication 1, caractérisés par le fait qu'ils sont pris, seuls ou en mélanges, dans le groupe constitué par :
- 2-(5,6,7,8-tetrahydro-5,5,8,8,-tetramethyl-2-naphthyl)-5-benzo(b)furanne carboxylate de méthyle,
- Acide 2-(5,6,7,8-tetrahydro-5,5,8,8,-tetramethyl-2-naphthyl)-5-benzo(b)furanne carboxylique,
- 2-(5,6,7,8-tetrahydro-5,5,8,8,-tetramethyl-2-naphthyl)-5-benzo(b)thiophene carboxylate de methyle,
- Acide 2-(5,6,7,8-tetrahydro-5,5,8,8,-tetramethyl-2-naphthyl)-5-benzo(b)thiophene carboxylique,
- Acide 2-(5,6,7,8-tetrahydro-5,5,8,8,-tetramethyl-2-naphthyl)-5-indole carboxylique,
- 2-(5,6,7,8-tetrahydro-5,5,8,8,-tetramethyl-2-naphthyl)-5-indole carboxylate de methyle,
- Acide 2-(1,2,3,4,4a,9b-Hexahydro-1,4a,9b-triméthyl-1,4-méthanodibenzofuran-8-yl)-benzo(b)thiophene-5-carboxylique,
- 2-(1,2,3,4,4a,9b-Hexahydro-1,4a,9b-triméthyl-1,4-méthanodibenzofuran-8-yl)-benzo(b)thiophene-5-carboxylate de methyle,
- Acide 2-(1,2,3,4,4a,9b-Hexahydro-1,4a,9b-triméthyl-1,4-méthanodibenzofuran-8-yl)-benzo(b)furanne-5-carboxylique ;
- Acide 2-(5,6,7,8-tetrahydro-5,5,8,8,-tetramethyl-3-methoxy-2-naphtyl)-5-benzo(b)furanne carboxylique,
- Acide 2-(5,6,7,8-tetrahydro-5,5,8,8,-tetramethyl-3-methoxy-2-naphtyl)-5-benzo(b)thiophene carboxylique,
- Acide 2-(5,6,7,8-tetrahydro-5,5,8,8,-tetramethyl-3-n-propyloxy-2-naphtyl)-5-benzo(b)thiophene carboxylique,
- Acide 2-(5,6,7,8-tetrahydro-5,5,8,8,-tetramethyl-3-n-propyloxy-2-naphtyl)-5-benzo(b)furanne carboxylique,
- Acide 2-(5,6,7,8-tetrahydro-5,5,8,8,-tetramethyl-3-n-heptyloxy-2-naphtyl)-5-benzo(b)furanne carboxylique,

9. Composés selon la revendication 1, caractérisés par le fait qu'ils présentent l'une au moins des, et de préférence toutes les, caractéristiques suivantes :
- R₁ est un radical -(CH₂)ₓ-CO-R₁₂ ou -(CH₂)ₓ-CO-O-R₁₃
- R₂ est un hydrogène
- Z₁ est un atome d'oxygène ou de soufre
- Ar est soit choisi parmi
le radical IV dans lequel :
- R₉ est un atome d'hydrogène
ou le radical III dans lequel :
- Z₂ est un radical C(R₇, R₈)
- n=2

10. Composés selon l'une quelconque des revendications précédentes pour une utilisation comme médicament.

11. Composés selon la revendication 10 pour une utilisation comme médicament destiné au traitement des affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle; pour traiter d'autres types de troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal); pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation; pour traiter toutes les proliférations dermiques ou épidermiques qu'elles soient bénignes ou malignes, qu'elles soient ou non d'origine virale telles que les verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires; pour traiter d'autres désordres dermatologiques tels que les dermatoses bulleuses et les maladies du collagène; pour traiter certains troubles ophtalmologiques, notamment les cornéopathies; pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique; pour prévenir ou guérir les stigmates de l'atrophie épidermique et/ou dermique induite par les corticostéroïdes locaux ou systémiques, ou tout autre forme d'atrophie cutanée, pour prévenir ou traiter les troubles de la cicatrisation ou pour prévenir ou réparer les vergetures; pour favoriser la cicatrisation, pour lutter contre les troubles de la fonction sébacée tels que l'hyperséborrhée de l'acné ou la séborrhée simple; pour le traitement ou la prévention des états cancéreux ou précancéreux, plus particuliérement les leucémies promyélocytaires; pour le traitement d'affections inflammatoires telles que l'arthrite, pour le traitement de toute affection d'origine virale au niveau cutané ou général; pour la prévention ou le traitement de l'alopécie; pour le traitement d'affections dermatologiques à composante immunitaire; pour le traitement d'affections du système cardiovasculaire telles que l'artériosclérose, pour le traitement de désordres cutanés dus à une exposition aux rayonnements U.V..

12. Composition pharmaceutique, caractérisée par le fait qu'elle comprend, dans un support pharmaceutiquement acceptable, au moins l'un des composés tels que définis à l'une quelconque des revendications 1 à 9.

13. Composition selon la revendication 12, caractérisée en ce que la concentration en composé(s) selon l'une des revendications 1 à 9 est comprise entre 0,001 % et 5 % en poids par rapport à l'ensemble de la composition.

14. Composition cosmétique, caractérisée par le fait qu'elle comprend, dans un support cosmétiquement acceptable, au moins l'un des composés tels que définis à l'une quelconque des revendications 1 à 9.

15. Composition selon la revendication 14, caractérisée en ce que la concentration en composé(s) selon l'une des revendications 1 à 9 est comprise entre 0,001 % et 3 % en poids par rapport à l'ensemble de la composition.

16. Utilisation d'une composition cosmétique telle que définie à l'une des revendications 14 ou 15 pour l'hygiène corporelle ou capillaire.
